(19) Europäisches Patentamt
European Patent Office
Office européen des brevets

(11) Veröffentlichungsnummer: **0 275 524 B1**

(12) **EUROPÄISCHE PATENTSCHRIFT**

(45) Veröffentlichungstag der Patentschrift: **15.05.91**

(51) Int. Cl.[5]: **C07D 333/08**, C07D 333/50, C07D 333/78

(21) Anmeldenummer: **87118933.8**

(22) Anmeldetag: **21.12.87**

(54) **Verfahren zur Herstellung von Thiophenen aus alpha-Methylenketonen im Eintopfverfahren und mittels des Verfahrens hergestellte neue anellierte Thiophene.**

(30) Priorität: **22.12.86 DE 3643983**

(43) Veröffentlichungstag der Anmeldung:
**27.07.88 Patentblatt 88/30**

(45) Bekanntmachung des Hinweises auf die Patenterteilung:
**15.05.91 Patentblatt 91/20**

(84) Benannte Vertragsstaaten:
**AT BE CH DE FR GB IT LI LU NL SE**

(56) Entgegenhaltungen:
**DE-A- 2 757 816**
**GB-A- 771 116**
**US-A- 2 658 903**
**US-A- 3 014 923**

**B. FRANCK et al.: "Liebigs Annalen der Chemie", Nr. 9, 1983, Seiten 1598-1606, Verlag Chemie GmbH, Weinheim, DE; F. BOBERG et al.: "Schwefelverbindungen des Erdöls, III - Dihydrodiindeno[b,d]thiophene"**

(73) Patentinhaber: **Von der Haar-Czogalla, Rita**
**Pater-Maier-Strasse 10**
**W-8152 Feldkirchen-Westerham(DE)**

(72) Erfinder: **Czogalla, Claus-Detleff, Dr.**
**Pater-Maier-Strasse 10**
**W-8152 Feldkirchen-Westerham(DE)**

(74) Vertreter: **Dr. Elisabeth Jung Dr. Jürgen Schirdewahn Dipl.-Ing. Claus Gernhardt**
**P.O. Box 40 14 68 Clemensstrasse 30**
**W-8000 München 40(DE)**

EP 0 275 524 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, Band 99, Nr. 17, 24. Oktober 1983, Seite 534, Zusammenfassung Nr. 139146q, Columbus, Ohio, US; O. GLINZER et al.: "Mass spectrometry of sulfur compounds as model substances for crude oil analysis - bisanellated thiophenes", & FRESENIUS' Z. ANAL. CHEM. 1983, 315(3), 208-12

MONATSHEFTE FÜR CHEMIE, Band 117, Nr. 5, Mai 1986, Seiten 621-629, Springer Verlag; C.R. NOE et al.: "Ein einfaches Verfahren zur Herstellung anellierter Thiophene"

CHEMICAL ABSTRACTS, Band 100, Nr. 13, 26. März 1984, Seite 627, Zusammenfassung Nr. 103098a, Columbus, Ohio, US; F. BOBERG et al.: "Sulfur compounds from petroleum. V. Dimethyl 3,5-diaryl-2,4-thiophenedicarboxylates from aryl methyl ketones", & PHOSPHORUS SULFUR 1983, 17(2), 135-40

## Beschreibung

Substituierte Thiophene und b,d-anellierte Thiophene sind für die Anwendung auf verschiedenen Gebieten von besonderem Interesse, insbesondere

- als Referenzmaterialien auf dem Gebiet der Analytik von Schwefelinhaltsstoffen fossiler Rohstoffe, wie Erdöle, Kohle, Kohleöle, Schieferöle und Teersande
- als Modellsysteme für Entschwefelungsstudien der oben genannten fossilen Rohstoffe, auch im technischen Maßstab
- als Oxidationsinhibitoren, zum Beispiel in Schmierstoffen
- als Wirkstoffe auf den Gebieten der Biozide und Pharmaka, sofern die Thiophene bestimmte Substituenten bzw. funktionelle Gruppen tragen
- als Monomerbausteine zur Herstellung von Polymeren, sofern die Thiophene bestimmte funktionelle Gruppen aufweisen.

Eine Zusammenfassende Darstellung der bisher üblichen, meist mehrstufigen Verfahren zur Herstellung solcher Thiophene findet sich in der Monographie von H.D. Hartough and S.L. Meisel "Compounds with condensed thiophene rings", Wiley (Interscience), New York, 1954.

Diese Verfahren gehen vielfach von aufwendig herzustellenden Ausgangsverbindungen aus oder von solchen, die unangenehme Eigenschaften haben, wie zum Beispiel Thiole, und die Ausbeuten lassen oft zu wünschen übrig. Einfache Thiophensynthesen, z.B. direkte Schwefelung entsprechender Ausgangsverbindungen oder Schwefelung mit $AlCl_3$-Zusatz oder Thermolyse von entsprechenden 1,2,3-Thiadiazolen, liefern nur geringe Ausbeuten und/oder isomere Gemische. Dieser Sachverhalt hat dazu geführt, daß solche Thiophene in der Herstellung sehr teuer sind, so daß nur die nachstehenden 5 Verbindungen erhältlich sind:

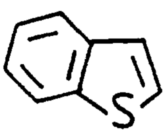
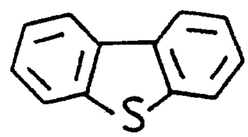
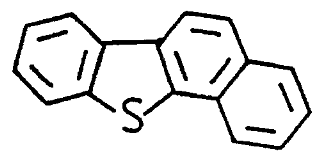

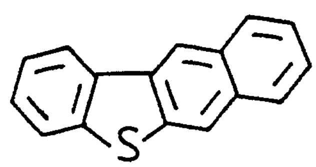
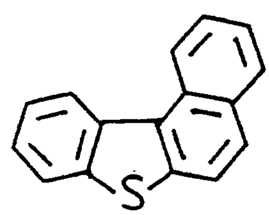

In einer Studie zur Synthese von Dihydrodiindenol(b,d)thiophenen, welche mögliche Inhaltsstoffe von Erdölen sind, mittels Thermolyse eine Indenothiadiazols werden z.B. nur Ausbeuten an 3 verschiedenen Verbindungen von 0.5 bis 1.0 bzw. 0.002 % erhalten. Auch bei Verwendung von Kupferpulver als Katalysator wurde nach 30stündigem Umsatz in siedendem tert.-Butylbenzol nur eine Mischung aus Verbindungen erhalten, in der lediglich eine Komponente in höherer Konzentration enthalten war (vgl. "Liebigs Ann.Chem.", 1983, S.1598-1607).

In "Phosphorus Sulfur", 1983, 17 (2), S.135-140, wird ferner über die Herstellung von Dimethyl-3,5-diaryl-2,4-thiophendicarboxylaten aus Dithiothionen berichtet, welche ihrerseits mittels einer zweistufigen Arbeitsweise aus Arylmethylketonen erhalten worden sind. Diese bekannten Arbeitsweise sind aber nicht zur Durchführung im industriellen Maßstab geeignet, sondern haben nur wissenschaftliches Interesse.

Aufgabe der Erfindung war es daher, eine Thiophensynthese zu finden, die die vorgenannten Nachteile nicht aufweist, hingegen gegenüber den vorbekannten Verfahren folgende Vorteile aufweist:

1) Die Thiophene sollen möglichst in einer einstufigen Synthese in guten Ausbeuten darstellbar sein;

2) Die Ausgangsmaterialien für die Thiophensynthese sollen einfach herzustellen sein oder käuflich erwerbbar sein und eine große Bandbreite innerhalb einer Stoffgruppe aufweisen;

3) Die Isolierung und Reindarstellung der Thiophene soll möglichst einfach sein und reine Produkte, auch in größeren Mengen, zum Beispiel für technische Studien, liefern.

Diese Aufgabe wird erfindungsgemäß gelöst.

Überraschenderweise wurde nämlich gefunden, daß bei der Umsetzung von α-Methylenketonen (über die Zwischenstufe der 2-Brom-1-ketone als Synthon) mit bekannten Schwefelungsmitteln, wie $P_4S_{10}$ oder Lawessons Reagenz (LR), Thiophene entstehen und zwar symmetrisch substituierte Thiophene bzw. b,d-anellierte Thiophene, wobei diese Synthese als Eintopfverfahren durchgeführt werden kann. Diese Umsetzungsmöglichkeit konnte nicht vorausgesehen werden, weil an sich die Bildung von 2-Brom-1-thioketonen als Endprodukte erwartet werden mußte.

Das erfindungsgemäße Verfahren zur Herstellung von substituierten Thiophenen und/oder b,d-anellierten Thiophenen ist daher dadurch gekennzeichnet, daß man ein gegebenenfalls durch eine oder mehrere lineare oder verzweigte Alkylgruppen, Methoxygruppen, maskierte Amino- oder Carbonylgruppen substituiertes α-Methylenketon aus der Gruppe der Arylmethylketone, hydroaromatischen 5-Ring-ketone, 6-Ringketone oder 7-Ringketone und der hydroheteroaromatischen Ketone in einem polaren Lösungsmittel mit Brom umsetzt, das Lösungsmittel abtrennt und dann das gebildete 2-Brom-1-keton direkt mit einem Schwefelungsmittel in einem Lösungsmittel unter Rückflußbedingungen bei Temperaturen bis 200 °C umsetzt und anschließend das gebildete Reaktionsprodukt in an sich bekannter Weise isoliert.

Schema 1

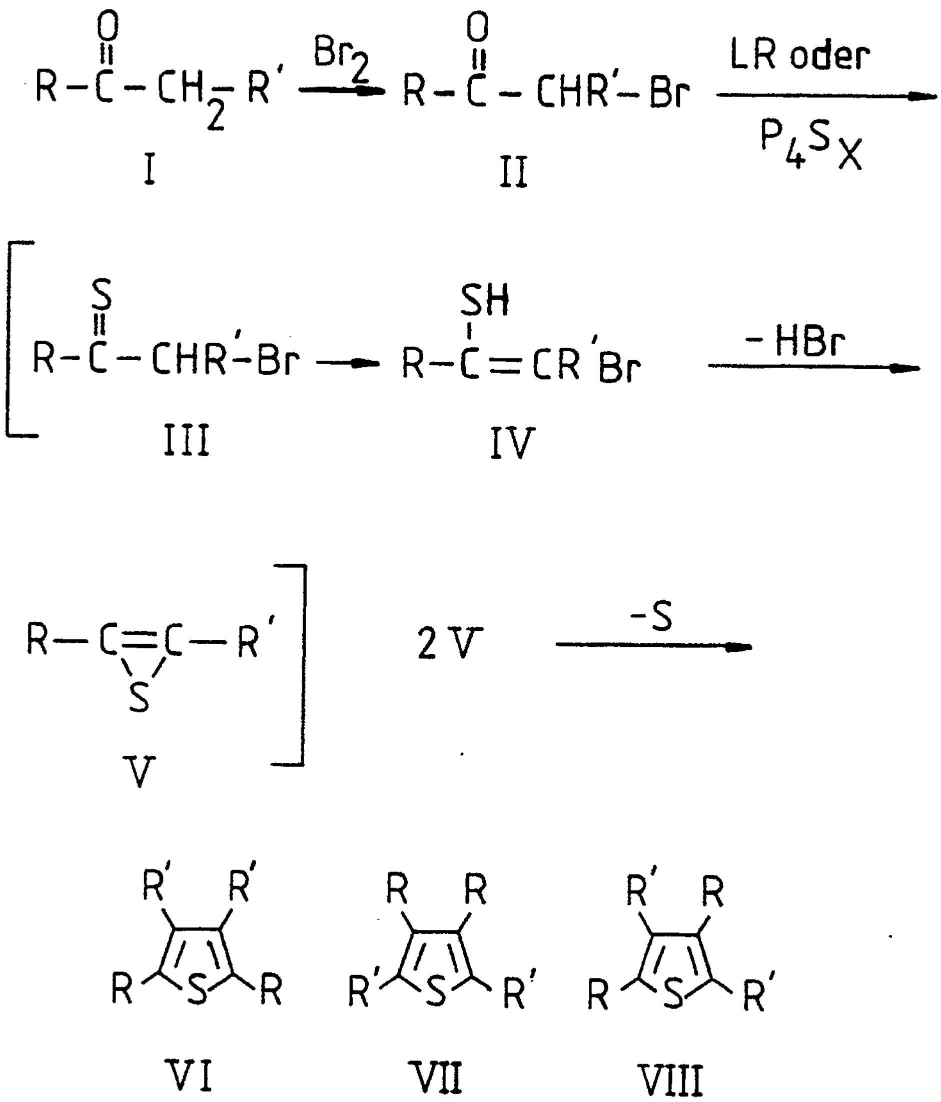


I, II, III, IV, V, VI, VII, VIII

R, R': Alkyl, Aryl, Cycloalkyl, Anellierte Systeme

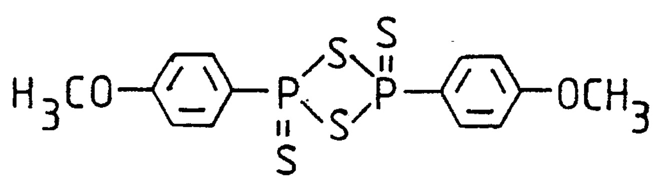

LR: LAWESSONS REAGENZ

Die erfindungsgemäße Thiophensynthese geht aus von leicht zugänglichen α-Methylenketonen vom Typ der Arylmethylketone, wie Acetophenone, oder von hydroaromatischen Fünfringketonen, Sechsringketonen oder Siebenringketonen, wie z.B. der 1-Indanone, 1-Acenaphthenone, 1-Tetralone, Tetrahydrobenzanthracenone, oder hydroheteroaromatischen Ketonen, wie Tetrahydrobenzothiophenone, oder von Benzosuberonen. Die Ketone als Ausgangsverbindungen der Thiophensynthese können auch substituiert sein. Als

Substituenten eignen sich z.B. lineare oder verzweigte Alkylgruppen, Methoxygruppen, maskierte Amino- und Carbonylgruppen.

Die eingesetzten Ketone werden in der Eintopfsynthese in an sich bekannter Weise in Polaren Lösungsmitteln, z.B. in Alkohlen, wie Methanol, mit $Br_2$ zu 2-Brom-1-ketonen umgesetzt. Andere geeignete polare Lösungsmittel sind Äther, Eisessig, Chloroform und Tetrahydrofuran. Besonders zweckmäßige Lösungsmittel sind niedrigmolekulare Alkohle mit 1 bis 4 Kohlenstoffatomen, z.B. Methanol oder Äthanol.

Nach Abtrennen des Lösungsmittels wird das gebildete Zwischenprodukt ohne weitere Reinigung mit einem an sich bekannten Schwefelungsmittel umgesetzt, bis die HBr-Entwicklung aufhört.

Es bilden sich die erfindungsgemäßen symmetrisch substituierten Thiophene, wie z.B. 2,5-Diphenylthiophene (aus Acetophenonen), oder symmetrisch anellierte Thiophene, wie z.B. Diindeno[b,d]thiophene (aus 1-Indanonen), alle von Typ VI (bgl. Schema 1).

Gemäß einer bevorzugten Ausführungsform verwendet man als Schwefelungsmittel eine Phosphorschwefelverbindung der Formel $P_4S_x$, in welcher x den Wert 3, 5, 7, 9 oder 10 hat, und als Lösungsmittel einen aromatischen Kohlenwasserstoff oder Pyridin. Geeignete aromatisch Kohlenwasserstoffe sind z.B. Benzol, Toluol und Xylol.

Sehr zweckmäßig wendet man dabei ein Molverhältnis von Schwefelungsmittel zu Keton im Bereich von 0,1:1 bis 1,5:1 an.

Gemäß einer weiteren bevorzugten Ausführungsform verwendet man als Schwefelungsmittel Lawessons Reagenz und als Lösungsmittel einen aromatischen Kohlenwasserstoff oder Pyridin. Auch hier sind besonders geeignete aromatische Kohlenwasserstoffe Benzol, Toluol und Xylol.

Sehr zweckmäßig wendet man dabei ein Molverhältnis von Schwefelungsmittel zu Keton im Bereich von 0,5:1 bis 1,5:1 an.

Man kann aber auch als Schwefelungsmittel eine Phosphorschwefelverbindung der Formel $P_4S_x$, in welcher x den Wert 3, 5, 7, 9 oder 10 hat, in Kombination mit $Na_2S$, $NaHCO_3$ oder $Na_2CO_3$ als Katalysator, und ein polares Lösungsmittel einsetzen. Hierfür geeignete polare Lösungsmittel sind Tetrahydrofuran, Diäthylenglykoldimethyläther oder Äther.

Weitere geeignete Schwefelungsmittel sind Siliciumdisulfid oder Borsulfid ($B_2S_3$), wobei man als Lösungsmittel zweckmässig Chloroform oder einen aromatischen Kohlenwasserstoff einsetzt, sowie 0,0-Diäthyldithiophosphorsäure.

Geeigneterweise führt man die Umsetzung mit dem Schwefelungsmittel unter Rückflußbedingungen bei Temperaturen bis 200 °C durch.

Beispiele für die auf diese Art und Weise hergestellten Thiophene zeigt die Tabelle I. Die in der Tabelle aufgeführten Thiophene Nr. 9 ($R_x = OCH_3$), 10, 12, 13 und 14 sind neu. Für die Thiophene Nr. 12, 13 und 14 konnte die angegebene Struktur noch nicht durch Röntgenstrukturanalyse bestätigt werden. Sie wird jedoch wegen der im Reaktionsschema angegebenen Abläufe der einzelnen Reaktionen als wahrscheinlich angesehen. Die physikalischen Kenndaten bestätigen außerdem die Existenz der Verbindungen als solcher.

Tabelle 1:

| α-Methylenketone | → | Thiophene v. Typ VI |
| --- | --- | --- |
| 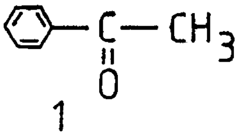 1 | | 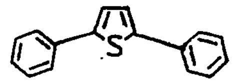 8 |
| 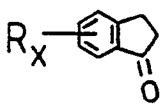 2a-2c | | 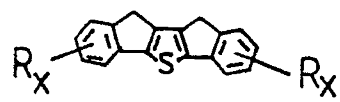 9a-9c |
| 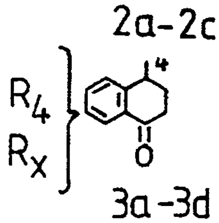 3a-3d | | 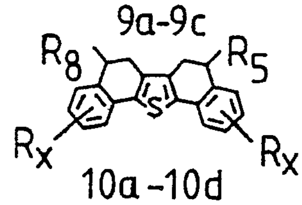 10a-10d |
| 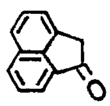 4 | | 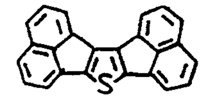 11 |
| 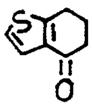 5 | | 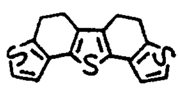 12 |
| 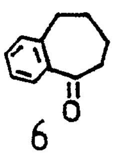 6 | | 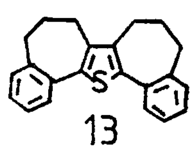 13 |
| 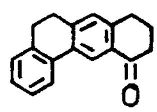 7 | | 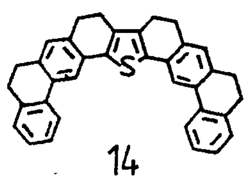 14 |

$R_X$: H, Alkyl, $OCH_3$

$R_4, R_5, R_8$: H, Alkyl

Die erfindungsgemäß herstellbaren Thiophene sind auch insofern von Interesse, als sie mittels an sich bekannter Verfahren zu gekuppelten Systemen, z.B. zu Biphenylen, entschwefelt werden können oder aber mit bestimmten Substituenten versehen werden können, die chromophore Eigenschaftan haben, und dann auf den Gebieten der Weißmacher und Farbstoffe Verwendung finden können.

Darüber hinaus lassen sich die erfindungsgemäß herstellbaren b,d-anellierten Thiophene mit bekannten

Verfahren zu den entsprechenden ungesättigten Verbindungen dehydrieren, so z.B.:

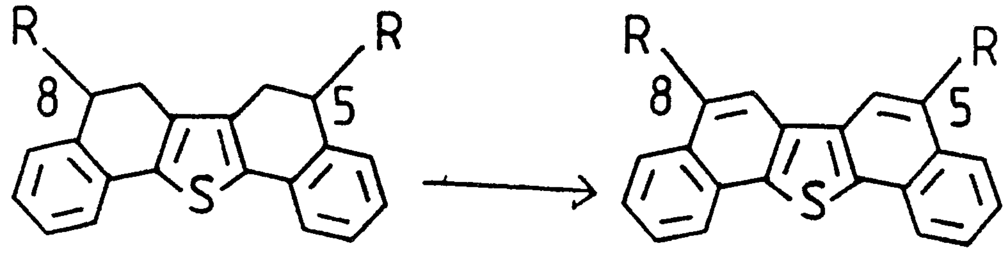

10a, R=H
10b, R=$CH_3$

15a, R=H
15b, R=$CH_3$

Die mit der neuen Thiophensynthese erzielbaren Vorteile gegenüber den Synthesen des Standes der Technik lassen sich wie folgt zusammenfassen:

- Große Bandbreite in bezug auf die herstellbaren Thiophensysteme, es lassen sich leicht Multiringsysteme aufbauen, so z.B. Verbindung 14 mit neun Ringen
- Herstellung in technischen Mengen, so z.B. Verb.10a, die leicht in Kg-Mengen synthetisiert werden kann.

Ein besonderes Merkmal der erfindungsgemäßen Thiophensynthese ist, daß statt der drei isomeren Thiophene, die nach Schema 1 als Gemisch theoretisch in gleichen Mengen anfallen können, in der Praxis nur das Thiophen VI in präparativ relevanten Mengen isoliert wird. Es fällt entweder aus der Reaktionslösung aus oder kann durch Säulenchromatographie leicht abgetrennt werden (Die Isomeren VII-VIII lassen sich nur mit der GC oder HPLC nachweisen, sofern Referenzsubstanzen zur Verfügung stehen).

Die erfindungsgemäß herstellbaren Thiophene lassen sich für verschiedene Bereiche einsetzen. Sie können dienen als:

- Referenzsubstanzen in der Analytik von Schwefelinhaltsstoffen fossiler Rohstoffe,
- Modellsysteme für Entschwefelungsstudien fossiler Rohstoffe, auch im technischen Maßstab,
- Oxidationsinhibitoren, z.B. in Schmierstoffen,
- Wirkstoffe auf dem Gebiet der Biozide und Pharmaka, sofern die Thiophensysteme bestimmte Substituenten bzw. bestimmte funktionelle Gruppen an den anellierten Ringen tragen,
- Monomerbausteine zur Herstellung von Polymeren mit bestimmten Eigenschaften, z.B. Hochtemperaturfestigkeit, sofern die Thiophene bestimmte Substituenten bzw. funktionelle Gruppen tragen.

Die nachstehenden Ausführungsbeispiele erläutern die Erfindung.

## Experimenteller Teil

Die 2-Brom-1-ketone werden nach der Methode von Hauptmann (vgl. S. Hauptmann, M. Scholz, I. Stopp und M. Stopp "Z.Chem.", 7, 351 (1967)) durch Bromierung der Ausgangsketone (0,025 Mol) unter Verwendung von 0,025 Mol Brom in Methanol dargestellt und werden nicht isoliert. Nach Beendigung der Reaktion rotiert man am Rotationskolbenverdampfer 1600 Pa (12 Torr) das Methanol ab, nimmt den Rückstand in trockenem Benzol auf, gibt Lawessons Reagenz (0,02 Mol) hinzu und kocht unter Rückfluß und unter Feuchtigkeitsausschluß bis zur Beendigung der HBr-Entwicklung. Man läßt das Reaktionsgemisch abkühlen, saugt den Niederschlag ab, wäscht mit Methanol und kristallisiert um. Wenn das Reaktionsprodukt nicht ausfällt, wird eingeengt und der Rückstand an 40 × 2 cm Kieselgel (65 g) mit Toluol, dann mit Cyclohexan/$CH_2Cl_2$(4:1) chromatographiert. Nach der üblichen Aufarbeitung wird umkristallisiert.

In der nachstehenden Tabelle II sind die Ausgangsketone, die hergestellten Thiophene mit ihren Schmelzpunkten, die Ausbeute sowie die Bruttoformel der betreffenden Verbindung, die Ergebnisse der Elementaranalyse und die Kennzeichnung durch Daten der $^1$H-NMR-Spektren und der IR-Spektren wiedergegeben.

Die Verbindungen Nr. 8, 9a und 11 sind bekannt und ihre strukturelle Identität wurde durch Vergleich

mit den Daten authentischer Verbindungen verifiziert (Mischschmelzpunkt, IR Spektren).

Die Verbindungen 10a und 10b wurden mittels Dehydrierung in die strukturähnlichen aromatischen Verbindungen umgewandelt und deren Struktur ermittelt.

Tabelle 2: Thiophene aus α-Methylenketonen

| | | Ausbeute |
|---|---|---|
| 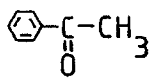 1 | 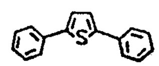 8 | 12% |
| 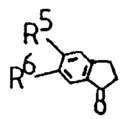 | 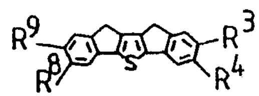 | |
| 2a: $R^5$, $R^6$ = H | 9a: $R^3$-$R^9$ = H | 50% |
| 2b: $R^5$ = $OCH_3$, $R^6$ = H | 9b: $R^3$, $R^9$ = $OCH_3$, $R^4$, $R^8$ = H | 40% |
| 2c: $R^5$, $R^6$ = $OCH_3$ | 9c: $R^3$-$R^9$ = $OCH_3$ | 23% |
| 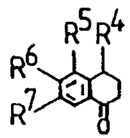 | 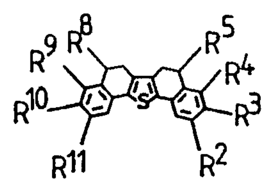 | |
| 3a: $R^4$-$R^7$ = H | 10a: $R^2$-$R^{11}$ = H | 53% |
| 3b: $R^4$ = $CH_3$ | 10b: $R^5$, $R^8$ = $CH_3$ | 50% |
| 3c: $R^6$ = $OCH_3$ | 10c: $R^3$, $R^{10}$ = $OCH_3$ | 51% |
| 3d: $R^5$, $R^7$ = CH | 10d: $R^2$, $R^4$, $R^9$, $R^{11}$ = $CH_3$ | 30% |

Fortset.: Tabelle 2: Physikalische Daten

| Verb. | Schm.punkt °C | Bruttoformel (Molmasse) | Elementaranalyse Ber. Gef. C | H | S | $^1$H-NMR ($CDCl_3$) δ = ppm | IR(KBr) ν = cm$^{-1}$ |
|---|---|---|---|---|---|---|---|
| 8 | 150-51 (Ethanol) | | | | | | 745 |
| 9a | 285 (Cumol) | | | | | | 750 |
| 9b | 290 (Toluol) | $C_{20}H_{16}O_2S$ (320.4) | 74.98<br>74.35 | 5.03<br>5.02 | 10<br>10.2 | | 1240 |
| 9c | 274 (Zers.) | $C_{22}H_{20}O_4S$ (380.4) | 69.45<br>71.79 | 5.30<br>5.69 | 8.48<br>9.11 | | 1250 |
| 10a | 230-32 (Toluol) | $C_{20}H_{16}S$ (288.4) | 83.29<br>83.01 | 5.59<br>5.47 | 11.12<br>11.03 | 2.5-3.2 (m; 8H, 4$CH_2$), 7.1-7.7 (m; 8$H_{arom}$) | 750 |
| 10b | 122-24 (i-Propanol) | $C_{22}H_{20}S$ (316.4) | 83.50<br>83.27 | 6.37<br>6.30 | 10.13<br>10.37 | 1.1-1.2 (d; 6H, 2$CH_3$), 2.15-3.5 (m; 6H, 2$CH_2$, 2CH), 7.1-7.7 (m; 8$H_{arom}$) | 757 |
| 10c | 222-24 (Toluol) | $C_{22}H_{20}O_2S$ (348.46) | | | 9.20<br>9.13 | 2.5-3.15 (m; 8H, 4$CH_2$), 3.8 (s; 6H, 2$OCH_3$), 6.65-7.5 (m; 6$H_{arom}$ | 1240 |
| 10d | 222-24 (Cyclohexan) | $C_{24}H_{24}S$ (344.6) | 83.66<br>83.51 | 7.02<br>7.48 | 9.32<br>9.19 | 2.32 (s; 12H, 4$CH_3$), 2.62-3.2 (m; 8H, 4$CH_2$), 6.8-7.4 (m; 4$H_{arom.}$ | 847 |

Fortsetzung Tabelle 2

| | | Ausbeute |
|---|---|---|
| 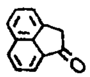 4 | 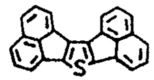 11 | 40% |
| 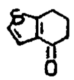 5 | 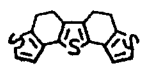 12 | 50% |
| 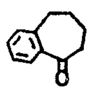 6 | 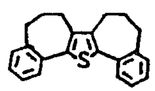 13 | 25% |
| 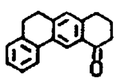 7 | 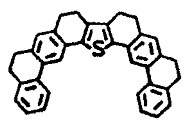 14 | 54% |

Fortsetzung Tabelle 2: Physikalische Daten

| Verb. | Schm.punkt °C | Bruttoformel (Molmasse) | Elementaranalyse C Ber. Gef. | H | S | $^1$H-NMR ($CDCl_3$) $\delta$=ppm | IR(KBr) $\nu$=$cm^{-1}$ |
|---|---|---|---|---|---|---|---|
| 11 | 278(Xylol) | | | | | | 757 |
| 12 | 254-56(Glyme) | $C_{16}H_{12}S_3$ (300.46) | 63.96<br>63.85 | 4.03<br>4.02 | 32.01<br>32.00 | 3.07-3.72 (m;8H,4$CH_2$),7.1-7.65(m;8$H_{arom.}$) | 808 |
| 13 | 159(Cyclohexan) | $C_{22}H_{20}S$ (316.4) | 83.50<br>83.03 | 6.37<br>6.39 | 10.13<br>10.38 | 2.0-2.86 (m;12H,6$CH_2$), 7.1-7.6(m;8$H_{arom.}$) | 753 |
| 14 | 282-85(Cumol) | $C_{36}H_{28}S$ (492.6) | 87.76<br>87.74 | 5.73<br>5.67 | 6.51<br>6.46 | 2.7-3.22 (m;16H,8$CH_2$), 7.0-8.0(m;12$H_{arom.}$ | 760 |

## Ansprüche

1. Verfahren zur Herstellung von substituierten Thiophenen und/oder b,d-anellierten Thiophenen, daher dadurch gekennzeichnet, daß man ein gegebenenfalls durch eine oder mehrere lineare oder verzweigte Alkylgruppen, Methoxygruppen, maskierte Amino- oder Carbonylgruppen substituiertes α-Methylenketon aus der Gruppe der Arylmethylketone, hydroaromatischen 5-Ring-ketone, 6-Ringketone oder 7-Ringketone und der hydroheteroaromatischen Ketone in einem polaren Lösungsmittel mit Brom umsetzt, das Lösungsmittel abtrennt und dann das gebildete 2-Brom-1-keton direkt mit einem Schwefelungsmittel in einem Lösungsmittel unter Rückflußbedingungen bei Temperaturen bis 200° C umsetzt und anschließend das gebildete Reaktionsprodukt in an sich bekannter Weise isoliert.

2. Verfahren nach Anspruch 1, dadurch gekennzeichnet, daß man als Arylmethylketon Acetophenon einsetzt.

3. Verfahren nach Anspruch 1, dadurch gekennzeichnet, daß man als hydroaromatisches Ringketon ein 1-Indanon, ein 1-Acenaphthenon, Tetrahydrobenzanthracenon oder ein 1-Tetralon oder ein Benzosuberon einsetzt.

4. Verfahren nach Anspruch 3, dadurch gekennzeichnet, daß man als hydroheteroaromatisches Keton ein Tetrahydrobenzothiophenon einsetzt.

5. Verfahren nach Anspruch 1 bis 4, dadurch gekennzeichnet, daß man als polares Lösungsmittel für die Umsetzung mit Brom einen Alkohol, Äther, Eisessig, Chloroform oder Tetrachlorkohlenstoff einsetzt.

6. Verfahren nach Anspruch 5, dadurch gekennzeichnet, daß man für die Umsetzung mit Brom einen niedrigmolekularen Alkohol mit 1 bis 4 Kohlenstoffatomen als Lösungsmittel einsetzt.

7. Verfahren nach Anspruch 6, dadurch gekennzeichnet, daß man Methanol als Lösungsmittel einsetzt.

8. Verfahren nach Anspruch 1 bis 7, dadurch gekennzeichnet, daß man als Schwefelungsmittel eine Phosphorschwefelverbindung der Formel $P_4S_x$, in welcher x den Wert 3, 5, 7, 9 oder 10 hat, und als Lösungsmittel einen aromatischen Kohlenwasserstoff oder Pyridin einsetzt.

9. Verfahren nach Anspruch 8, dadurch gekennzeichnet, daß man für die Umsetzung mit dem Schwefelungsmittel Benzol, Toluol und/oder Xylol als Lösungsmittel einsetzt.

10. Verfahren nach Anspruch 8 und 9, dadurch gekennzeichnet, daß man ein Molverhältnis von Schwefelungsmittel zu Keton im Bereich von 0,1:1 bis 1,5:1 anwendet.

11. Verfahren nach Anspruch 1 bis 7, dadurch gekennzeichnet, daß man als Schwefelungsmittel Lawessons Reagenz und als Lösungsmittel einen aromatischen Kohlenwasserstoff oder Pyridin einsetzt.

12. Verfahren nach Anspruch 11, dadurch gekennzeichnet, daß man für die Umsetzung mit dem Schwefelungsmittel Benzol, Toluol und/oder Xylol als Lösungsmittel einsetzt.

13. Verfahren nach Anspruch 11 und 12, dadurch gekennzeichnet, daß man ein Molverhältnis von Schwefelungsmittel zu Keton im Bereich von 0,5:1 bis 1,5:1 anwendet.

14. Verfahren nach Anspruch 1 bis 7, dadurch gekennzeichnet, daß man als Schwefelungsmittel eine Phosphorschwefelverbindung der Formel $P_4S_x$, in welcher x den Wert 3, 5, 7, 9 oder 10 hat, in Kombination mit $Na_2S$, $NaHCO_3$ oder $Na_2CO_3$ als Katalysator, und ein polares Lösungsmittel einsetzt.

15. Verfahren nach Anspruch 14, dadurch gekennzeichnet, daß man als polares Lösungsmittel Tetrahydrofuran, Diäthylenglykoldimethyläther oder Äther einsetzt.

16. Verfahren nach Anspruch 1 bis 7, dadurch gekennzeichnet, daß man als Schwefelungsmittel Siliciumdisulfid oder Borsulfid ($B_2S_3$) und als Lösungsmittel Chloroform oder einen aromatischen Kohlenwasserstoff einsetzt.

17. Verfahren nach Anspruch 1 bis 7, dadurch gekennzeichnet, daß man als Schwefelungsmittel 0,0-Diäthyldithiophosphorsäure einsetzt.

**18.** Verbindungen der nachstehenden Formel

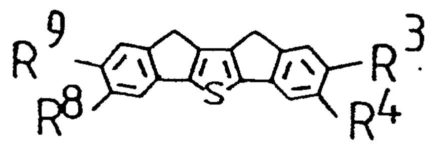

in der
a) $R^4$ und $R^8$ jeweils Wasserstoff und $R^3$ und $R^9$ jeweils die Gruppe $-OCH_3$ sind;
b) $R^3$, $R^4$, $R^8$ und $R^9$ jeweils die Gruppe $-OCH_3$ sind.

**19.** Verbindungen der nachstehenden Formel

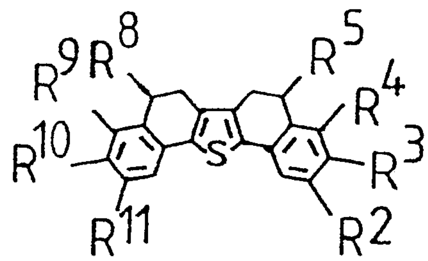

in welcher
a) alle R Wasserstoff bedeuten;
b) $R^5$ und $R^8$ jeweils die Gruppe $-CH_3$ und alle übrigen R Wasserstoff bedeuten;
c) $R^3$ und $R^{10}$ jeweils die Gruppe $-OCH_3$ und alle übrigen R Wasserstoff bedeuten;
d) $R^2$, $R^4$, $R^9$ und $R^{11}$ jeweils die Gruppe $-CH_3$ und alle übrigen R Wasserstoff bedeuten.

**20.** Verbindung, herstellbar aus 4-Oxo-4,5,6,7-tetrahydrobenzo(b)-thiophen nach dem Verfahren gemäß Anspruch 1.

**21.** Verbindung, herstellbar aus Benzosuberon (6, 7, 8, 9-tetrahydro-5H-benzocyclohepten-5-on) nach dem Verfahren gemäß Anspruch 1.

**22.** Verbindung, herstellbar aus 5, 6, 8, 9-Tetrahydrobenz(a)-anthracen-11-on nach dem Verfahren gemäß Anspruch 1.

## Claims

**1.** Process for the preparation of substituted thiophenes and/or b,d-fused thiophenes, characterised in that an α-methylene ketone, which is optionally substituted by one or more linear or branched alkyl groups, methoxy groups, or masked amino or carboxyl groups, from the group comprising aryl methyl ketones, hydroaromatic 5-membered ring ketones, 6-membered ring ketones or 7-membered ring ketones and hydroheteroaromatic ketones is reacted with bromine in a polar solvent, the solvent is removed and then the 2-bromo-1-ketone formed is reacted directly with a sulphurising agent in a solvent under reflux conditions at temperatures up to 200 °C and the reaction product formed is then isolated in a manner known per se.

**2.** Process according to Claim 1, characterised in that acetophenone is employed as the aryl methyl ketone.

**3.** Process according to Claim 1, characterised in that a 1-indanone, a 1-acenaphthenone, tetrahydrobenzanthracenone or a 1-tetralone or a benzosuberone is employed as the hydroaromatic ring ketone.

4. Process according to Claim 3, characterised in that a tetrahydrobenzothiophenone is employed as the hydroheteroaromatic ketone.

5. Process according to Claims 1 to 4, characterised in that an alcohol, ether, glacial acetic acid, chloroform or carbon tetrachloride is employed as the polar solvent for the reaction with bromine.

6. Process according to Claim 5, characterised in that a low molecular weight alcohol having 1 to 4 carbon atoms is employed as the solvent for the reaction with bromine.

7. Process according to Claim 6, characterised in that methanol is employed as the solvent.

8. Process according to Claims 1 to 7, characterised in that a phosphorus-sulphur compound of the formula $P_4S_X$ in which x has the value 3, 5, 7, 9 or 10 is employed as the sulphurising agent and an aromatic hydrocarbon or pyridine is employed as the solvent.

9. Process according to Claim 8, characterised in that benzene, toluene and/or xylene is are employed as the solvent for the reaction with the sulphurising agent.

10. Process according to Claims 8 and 9, characterised in that a molar ratio of sulphurising agent to ketone in the range from 0.5:1 to 1.5:1 is used.

11. Process according to Claims 1 to 7, characterised in that Lawesson's reagent is employed as the sulphurising agent and an aromatic hydrocarbon or pyridine is employed as the solvent.

12. Process according to Claim 11, characterised in that benzene, toluene and/or xylene is employed as the solvent for the reaction with the sulphurising agent.

13. Process according to Claims 11 and 12, characterised in that a molar ratio of sulphurising agent to ketone in the range from 0.5:1 to 1.5:1 is used.

14. Process according to Claims 1 to 7, characterised in that a phosphorus-sulphur compound of the formula $P_4S_X$ in which x has the value 3, 5, 7, 9 or 10 is employed as the sulphurising agent, in combination with $Na_2S$, $NaHCO_3$ or $Na_2CO_3$ as a catalyst, and a polar solvent.

15. Process according to Claim 14, characterised in that tetrahydrofuran, diethylene glycol dimethyl ether or ether is employed as the polar solvent.

16. Process according to Claims 1 to 7, characterised in that silicon disulphide or boron sulphide ($B_2S_3$) is employed as the sulphurising agent and chloroform or an aromatic hydrocarbon is employed as the solvent.

17. Process according to Claims 1 to 7, characterised in that 0,0-diethyldithiophosphoric acid is employed as the sulphurising agent.

18. Compounds of the formula below

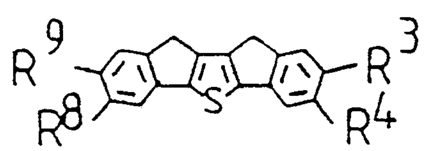

in which

a) $R^4$ and $R^8$ are in each case hydrogen and $R^3$ and $R^9$ are in each case the group $-OCH_3$; and
b) $R^3$, $R^4$, $R^8$ and $R^9$ are in each case the group $-OCH_3$.

**19.** Compounds of the formula below

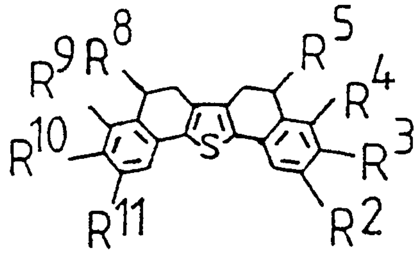

in which

a) all R groups denote hydrogen;
b) $R^5$ and $R^8$ in each case denotes the group $-CH_3$ and all other R groups denote hydrogen;
c) $R^3$ and $R^{10}$ in each case denote the group $-OCH_3$ and all other R groups denote hydrogen;
d) $R^2$, $R^4$, $R^9$ and $R^{11}$ in each case denote the group $-CH_3$ and all other R groups denote hydrogen.

**20.** Compound, preparable from 4-oxo-4,5,6,7-tetrahydrobenzo(b)-thiophene by the process according to Claim 1.

**21.** Compound, preparable from benzosuberone (6,7,8,9-tetrahydro-5H-benzocyclohepten-5-one) by the process according to Claim 1.

**22.** Compound, preparable from 5,6,8,9-tetrahydrobenz(a)-anthracen-11-one by the process according to Claim 1.

## Revendications

**1.** Procédé pour la fabrication de thiophènes et/ou de thiophènes b,d-condensés substitués, caractérisé en ce que l'on fait réagir avec le brome une α-méthylènecétone éventuellement substituée par un ou plusieurs groupes alkyles linéaires ou ramifiés, groupes méthoxy, groupes amino ou carbonyles masqués, du groupe des arylméthylcétones, des cétones hydroaromatiques à 5, 6 ou 7 chaînons et des cétones hydrohétéroaromatiques, dans un solvant polaire, on sépare le solvant et ensuite on fait réagir directement la 2-bromo-1-cétone formée avec un agent de sulfuration dans un solvant au reflux à des températures allant jusqu'à 200 °C et ensuite on isole de manière connue le produit de réaction formé.

**2.** Procédé selon la revendication 1, caractérisé en ce que l'on utilise comme arylméthylcétone l'acétophénone.

**3.** Procédé selon la revendication 1, caractérisé en ce que l'on utilise comme cétone cyclique hydroaromatique une 1-indanone, une 1-acénaphténone, la tétrahydrobenzanthracénone ou une 1-tétralone ou une benzosubérone.

**4.** Procédé selon la revendication 3, caractérisé en ce que l'on utilise comme cétone hydrohétéroaromatique une tétrahydrobenzothiophénone.

**5.** Procédé selon l'une des revendications 1 à 4, caractérisé en ce que l'on utilise comme solvant polaire pour la réaction avec le brome un alcool, l'éther, l'acide acétique, le chloroforme ou le tétrachlorure de carbone.

**6.** Procédé selon la revendication 5, caractérisé en ce que l'on utilise comme solvant pour la réaction avec le brome un alcool de bas poids moléculaire en $C_1$-$C_4$.

**7.** Procédé selon la revendication 6, caractérisé en ce que l'on utilise comme solvant le méthanol.

**8.** Procédé selon l'une des revendications 1 à 7, caractérisé en ce que l'on utilise comme agent de sulfuration un composé soufré du phosphore de formule $P_4S_X$, dans laquelle x a la valeur 3, 5, 7, 9 ou 10, et comme solvant un hydrocarbure aromatique ou la pyridine.

**9.** Procédé selon la revendication 8, caractérisé en ce que l'on utilise comme solvant pour la réaction avec l'agent de sulfuration le benzène, le toluène et/ou le xylène.

**10.** Procédé selon les revendications 8 et 9, caractérisé en ce que l'on utilise un rapport molaire de l'agent de sulfuration à la cétone dans l'intervalle de 0,1 : 1 à 1,5 : 1.

**11.** Procédé selon les revendications 1 à 7, caractérisé en ce que l'on utilise comme agent de sulfuration le réaction de Lawesson et comme solvant un hydrocarbure aromatique ou la pyridine.

**12.** Procédé selon la revendication 11, caractérisé en ce que l'on utilise comme solvant pour la réaction avec l'agent de sulfuration le benzène, le toluène et/ou le xylène.

**13.** Procédé selon les revendications 11 et 12, caractérisé en ce que l'on applique un rapport molaire de l'agent de sulfuration à la cétone de 0,5 : 1 à 1,5 : 1.

**14.** Procédé selon les revendications 1 à 7, caractérisé en ce que l'on utilise comme agent de sulfuration un composé soufré du phosphore de formule $P_4S_X$, dans laquelle x a la valeur 3, 5, 7, 9 ou 10, en combinaison avec $Na_2S$, $NaHCO_3$ ou $Na_2CO_3$ comme catalyseur et un solvant polaire.

**15.** Procédé selon la revendication 14, caractérisé en ce que l'on utilise comme solvant polaire le tétrahydrofuranne, l'éther diméthylique de diéthylèneglycol ou l'oxyde de diéthyle.

**16.** Procédé selon les revendications 1 à 7, caractérisé en ce que l'on utilise comme agent de sulfuration le disulfure de silicium ou le sulfure de bore ($B_2S_3$) et comme solvant le chloroforme ou un hydrocarbure aromatique.

**17.** Procédé selon les revendications 1 à 7, caractérisé en ce que l'on utilise comme agent de sulfuration le dithiophosphate de 0,0-diéthyle.

**18.** Composés de formule suivante :

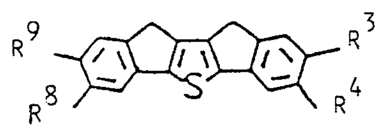

dans laquelle

a) $R^4$ et $R^8$ sont chacun l'hydrogène et $R^3$ et $R^9$ sont chacun le groupe $-OCH_3$;

b) $R^3$, $R^4$, $R^8$ et $R^9$ sont chacun le groupe $-OCH_3$.

**19.** Composés de formule suivante :

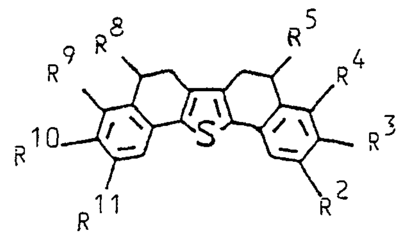

dans laquelle

a) tous les restes R représentent l'hydrogène ;
b) $R^5$ et $R^8$ représentent chacun le groupe $-CH_3$ et les autres restes R l'hydrogène ;
c) $R^3$ et $R^{10}$ représentent chacun le groupe $-OCH_3$ et les autres restes R l'hydrogène ;
d) $R^2$, $R^4$, $R^9$ et $R^{11}$ représentent chacun le groupe $-CH_3$ et les autres restes R l'hydrogène.

**20.** Composé préparable par le procédé selon la revendication 1 à partir du 4-oxo-4,5,6,7-tétrahydrobenzo-(b)-thiophène.

**21.** Composé préparable par le procédé selon la revendication 1 à partir de la benzosubérone (6,7,8,9-tétrahydro-5H-benzocycloheptène-5-one).

**22.** Composé préparable par le procédé selon la revendication 1 à partir de la 5,6,8,9-tétrahydrobenzo(a)-anthracène-11-one.